# EUROPEAN PATENT SPECIFICATION

(11) **EP 2 716 771 B1**
(45) Date of publication and mention of the grant of the patent: **06.01.2016**
(21) Application number: 12790104.9
(22) Date of filing: 21.05.2012
(51) Int. Cl.: C13B 30/00, C12P 19/12

(54) **METHOD FOR PRODUCING SOLID MATERIAL FROM SACCHARIDE SOLUTION, AND SOLID MATERIAL**
VERFAHREN ZUR HERSTELLUNG EINES FESTSTOFFS AUS EINER SACCHARIDLÖSUNG UND FESTSTOFF
PROCÉDÉ DE FABRICATION D'UNE MATIÈRE SOLIDE À PARTIR D'UNE SOLUTION DE SACCHARIDE ET MATIÈRE SOLIDE

(30) Priority: 23.05.2011 JP 2011114934; 28.11.2011 JP 2011259313
(43) Date of publication of application: 09.04.2014
(73) Proprietor: Mitsui Sugar Co., Ltd., Chuo-ku Tokyo 103-8423 (JP); San-ei Sucrochemical Co., Ltd., Chita-shi, Aichi 478-8503 (JP)
(72) Inventor: SUGITANI Toshiaki, Chigasaki-shi Kanagawa 253-0042 (JP); MIYASAKA Kiyoaki, Chigasaki-shi Kanagawa 253-0042 (JP); HIRAOKA Takeshi, Chita-shi Aichi 478-8503 (JP); NARITA Yasushi, Chita-shi Aichi 478-8503 (JP)
(74) Representative: Nony
(86) International application number: PCT/JP2012/062962
(87) International publication number: WO 2012/161165

(56) References cited:
- WO-A1-98/53089
- JP-A- 1 124 350
- JP-A- 2 257 888
- JP-A- 5 130 886
- JP-A- 9 019 300
- JP-A- 56 117 796
- JP-A- 2002 142 690
- JP-A- 2004 283 026
- JP-A- 2009 530 356
- JP-A- 2009 536 168

## Description

### FIELD OF THE INVENTION

The present invention relates to a process for producing a solid material from a saccharide solution.

### BACKGROUND OF THE INVENTION

Isomaltulose is a disaccharide which is obtained by subjecting sucrose to action of an alpha-glucosyltransferase which is an enzyme produced by Protaminobacter rubrum, Serratia plymuthica, Erwinia rhapontici, or Klebsiella sp., so that an alpha-1,2 bond in sucrose is changed into an alpha-1,6 bond.

Saccharide composition of a saccharide solution obtained by subjecting a sucrose solution to action of the enzyme is isomaltulose of 60 to 90 mass%, trehalulose of 5 to 35 mass%, glucose of 0.2 to 5 mass%, and fructose of 0.2 to 5 mass%. The saccharide solution is concentrated so that isomaltulose is crystallized to obtain isomaltulose crystals in the solution (crystallization step) and, then, the obtained crystals are collected by centrifugation (centrifugation step). In this way, crystallized isomaltulose is obtained by subjecting the saccharide solution to the crystallization step and the centrifugation step. Crystallized isomaltulose is sold as crystalline Palatinose (trademark) IC (Mitsui Sugar Co., Ltd., an isomaltulose purity of 99.0% or more). Meanwhile, saccharide composition of a syrup obtained in the centrifugation step is trehalulose of 53 to 59% and isomaltulose of 11 to 17%. This syrup is sold as Palatinose (trademark) syrup-ISN (Mitsui Sugar Co., Ltd.).

A solubility of isomaltulose in water is low and, therefore, isomaltulose is easily crystallized. On the other hand, trehalulose does not crystallize and remains as liquid. Therefore, the centrifugation step is essential for removing trehalulose to sell the separated isomaltulose as a solid product or a powdery product. In the centrifugation step, the crystallized isomaltulose and the syrup are obtained in a certain proportion. However, a balance of the demand and supply for the syrup does not match sometimes, therefore, the syrup is left over.

Patent Literature No.1 describes a process for producing a saccharide formulation, wherein the method is characterized in that, in converting sucrose to Palatinose by a bacterial enzyme which converts sucrose to Palatinose, the amounts of glucose and fructose generated in the conversion are controlled by changing temperature, so that the saccharides generated in the conversion are solidified all together (claims). A means for solidifying the saccharides all together may be a method of solidification and pulverization, a spray-drying method, a method of vacuum-drying in a drum, or a foam-drying method (page 2, lower left column, lines 10 to 13). In the spray-drying method, a concentrated saccharide solution or a saccharide solution in a massecuite form and the already-powderized saccharide formulation of the invention of Patent literature No.1 are each separately made into thin layers by centrifugal force, and these two layers are collided with each other in an intersecting manner to be granulated and solidified in a solidification gas (page 2, lower left column, line 17 to lower right column, line 1).

Patent Literature No.2 describes granules comprising lactose and starch (claim 1). The granules are obtained in a method comprising a step of spray-drying a suspension of lactose and starch (paragraph 0036). Before the spray-drying, a starch suspension is prepared in cold water, to which lactose monohydrate is added (paragraph 0037). The mixture, which is usually at 15 to 25 degrees Celsius, is spray-dried at an inlet temperature of about 160 degrees Celsius and at such a flow rate that an air temperature and a temperature of the spray-dried product at an outlet are about 65 degrees Celsius in a routinely-used spray-dryer known to a person having ordinary skill (paragraph 0037).

Patent Literature No.3 describes a process for producing powdery glucose, characterized in that starch is hydrolyzed by acid or an enzyme, the hydrolysate is optionally purified in a routine method, and concentrated to form a massecuite with or without adding crystal seeds, and the massecuite is spray-dried to produce powdery glucose (claims).

Patent Literature No.4 describes a process for producing a powder of mono- or di-saccharide comprising a step of spray-drying a massecuite of saccharified solution comprising maltose or glucose, characterized in that, after the spray-drying step, an aging step is carried out in which an ambient relative humidity (ambient RH) is adjusted to an equilibrium relative humidity (equilibrium RH) corresponding to a Bx value (Brix: saccharide concentration) of a syrup film (mother solution adherent to the spray-dried product) of 82 ± 2 (claim 1).

Patent Literature Nos.5 to 7 describe a method for spray-drying a solution containing oligosaccharide.

Patent Literature No.5 describes a process for producing powder of saccharide mainly consisting of oligosaccharides (hereinafter, referred to as "oligosaccharide") from an aqueous solution of maltose and oligosaccharides having molecular weights equal to or larger than that of maltotriose (claim 1). The process comprises a drying step in which a concentrated aqueous solution obtained in a concentration step is sprayed and brought into contact with a hot air to powderize the oligosaccharide (claim 1). An inlet temperature of the hot air in the drying step is 80 to 200, preferably 100 to 160 degrees Celsius (page 5, lower left column, lines 5 to 6).

Patent Literature No.6 describes a process for producing powdery maltose (claim 1) . The process is characterized in that a highly pure maltose solution obtained by enzymatically hydrolyzing a solution of liquidized starch having a low hydrolysis degree and containing maltose of 90% or more and maltotriose of 2.5% or less is concentrated to a solid content of 65 to 80%, to which seed crystals are added; primary crystals are formed at 25 ± 5 degrees Celsius to a crystallization ratio of 50 ± 5%; an appropriate amount of a maltose solution is, if necessary, added so that a viscosity of the solution at a temperature in the crystallization step is 70000 centipoise or less; the maltose solution is spray-dried to obtain powder having a water content of 5.5 to 7.5%; and the powder is immediately subjected to an aging step in an atmosphere at a high temperature and a high humidity, i.e., a relative humidity of 50 to 70% and an absolute humidity of 45 to 185 g water/kg dry air to cause crystallization and drying (claim 1).

Patent Literature No.7 describes a process for producing a non-hygroscopic powdery composition, wherein the process comprises a step of spray-drying, without any carrier for spray-drying, an aqueous solution comprising at least one material which is inherently hygroscopic and has a glass transition temperature of 10 to 110 degrees Celsius, and a low temperature fluid, especially a food-grade low temperature fluid, or a mixture of low temperature fluids, especially those selected from carbon dioxide, nitrogen gas, and liquidized air (claim 1). The aqueous solution used in the process is obtained by dissolving the low temperature fluid into a starting aqueous solution comprising the material which is inherently hygroscopic (claim 1).

The following Patent Literature Nos. 8 to 11 describe that a solution containing sugar alcohol is spray-dried.

Patent Literature No. 8 describes a composition obtainable by co-spray-drying, wherein the composition comprises mannitol in an amount of less than 10 mass% and consists substantively of two or more of polyols (claim 1). The composition is obtainable by dissolving two or more polyols in water and spraying the resulting aqueous mixture in an air flow having a temperature of 120 to 300 degrees Celsius (claim 2).

Patent Literature No. 9 describes the use of spray-dried powder comprising sugar alcohol for preventing an active ingredient from being decomposed or denatured by compression or for preventing a function of a functional particle from being altered by compression in the production of a drug by compression (claim 1). Patent Literature No. 10 describes an aid for producing tablet by direct compression, characterized in that the aid comprises xylitol in an amount of more than 90 mass% and one or more other polyols in an amount of less than 10 mass% and that the aid is produced by spray-drying or granulation in a fluidized bed (claim 1).

Patent Literature No. 11 describes a process for producing crystalline maltitol, and syrup-comprising maltitol crystals (claim 1). The method is characterized in that the following steps are consecutively conducted: 1) a first step in which a syrup having a concentration of 30 to 75 wt% where 81 to 90 wt% of a solid content is maltose is catalytically hydrogenated to obtain a corresponding sugar alcohol syrup, 2) a second step in which the sugar alcohol syrup is fed to a tower packed with a cation exchange resin to chromatographically obtain a maltitol-rich syrup fraction in which the solid content comprises maltitol of 92 to 99.9 wt%, and 3) a third step in which, after the maltitol-rich syrup fraction is concentrated, a part of the concentrated syrup is crystallized in the presence of seed crystals to collect crystalline maltitol, and the remaining part of the concentrated syrup is spray-dried or cooled and kneaded in the presence of seed crystals to obtain syrup-comprising maltitol crystals (claim 1).

The specification of the Japanese patent application No. 2011-27216 describes a process for producing a solid material from a saccharide solution comprising isomaltulose, wherein a sucrose solution is subjected to action of an enzyme which produces isomaltulose from sucrose to prepare a saccharide solution comprising isomaltulose. The process comprises a step of heating the saccharide solution to adjust a solid content of the saccharide solution to 77-96 mass%, a step of subjecting the resulting one to a shearing force to generate crystal nuclei while keeping the temperature at 65 to 120 degrees Celsius, followed by cooling the sheared product to obtain the solid material (claim 1).
Patent Literature No.1: Japanese Patent Application Laid-Open No. SHO-56-117796
Patent Literature No.2: Japanese Patent Application Laid-Open No. 2002-142690
Patent Literature No.3: Japanese Patent Application Laid-Open No. SHO-39-4834
Patent Literature No.4: Japanese Patent Application Laid-Open No. 2004-283026
Patent Literature No.5: Japanese Patent Application Laid-Open No. SHO-61-93129
Patent Literature No.6: Japanese Patent Application Laid-Open No. SHO-60-92299
Patent Literature No.7: Japanese National Phase Publication No. 2009-530356
Patent Literature No.8: Japanese National Phase Publication No. HEI-09-507863
Patent Literature No.9: WO2002/070013
Patent Literature No.10: Japanese National Phase Publication No. 2001-519378
Patent Literature No.11: Japanese Patent Application Laid-Open No. HEI-09-19300

### SUMMARY OF THE INVENTION

### PURPOSE OF THE INVENTION

As described above, the saccharide composition of the isomaltulose-containing saccharide solution obtained by subjecting a sucrose solution to action of an enzyme, alpha-glucosyltransferase, is isomaltulose of 60 to 90 mass%, trehalulose of 5 to 35 mass%, glucose of 0.2 to 5 mass% and fructose of 0.2 to 5 mass%. Trehalulose is non-crystalline, i.e. liquid. Therefore, without a centrifugation step, it is difficult to obtain a solid material from the isomaltulose-containing saccharide solution, because of a non-crystalline saccharide solution consisting mainly of trehalulose. Accordingly, the isomaltulose-containing saccharide solution itself could not be sold as a solidified or powderized isomaltulose product. As a conventional solidified or powderized isomaltulose product, the above-mentioned crystalline Palatinose IC (Mitsui Sugar Co., Ltd.) may be mentioned. This product is isomaltulose crystals. In order to obtain isomaltulose crystals, the crystallization step and the centrifugation step were required, as above-described. The centrifugation step is essential especially for separating isomaltulose crystals from the non-crystalline saccharide solution. In contrast, isomaltulose accounts for about 80 mass% of the saccharides in the saccharide solution obtained by subjecting a sucrose solution to action of alpha-glucosyltransferase. Therefore, if the saccharide solution itself can be solidified or powderized, such a solidified or powderized product can be sold as an isomaltulose product, though its isomaltulose purity is lower than that of conventional ones. A purpose of the present invention is to provide a process for producing an isomaltulose product which can be sold as a solid product or a powdery product, without the aforesaid centrifugation step. Another purpose of the present invention is to provide a solid material comprising the aforesaid non-crystalline saccharide solution.

### MEANS FOR ACHIEVING THE PURPOSES

The present invention provides a process for producing an isomaltulose-containing solid material from a saccharide solution comprising isomaltulose, wherein said solid material comprises isomaltulose in an amount of 70 to 90 mass% and trehalulose in an amount of 8 to 25 mass %, wherein a sucrose solution is subjected to action of an enzyme which produces isomaltulose from sucrose to prepare a saccharide solution comprising isomaltulose. This process comprises (a) a step of causing isomaltulose to crystalize to obtain crystals having a median diameter of 5 to 60 µm in the saccharide solution, and (b) a step of spray-drying the saccharide solution containing the isomaltulose crystals at an air temperature of 50 to 95 degrees Celsius to obtain the solid material. The present invention also provides a solid material comprising isomaltulose of an amount of 70 to 90 mass% and trehalulose in an amount of 8 to 25 mass %, wherein the solid material is in a spherical form and has a median diameter of 60 to 300 µm, as determined by laser diffraction particle size distribution measurement.

### EFFECTS OF THE INVENTION

According to the process of the present invention, a saccharide solution obtained by subjecting a sucrose solution to action of an enzyme which produces isomaltulose from sucrose is solidified without the previous centrifugation step. Thus, without centrifuging the saccharide solution, the saccharide solution, which comprises a non-crystalline substance such as trehalulose, is solidified as a whole. Consequently, an isomaltulose-rich solid material is obtained.

The isomaltulose-containing solid material obtained by the present process is not sticky but dry, especially powdery. Consequently, the solid material can be sold as a solid isomaltulose product, especially as a powdery isomaltulose product.

The isomaltulose-containing solid material obtained by the present process is white. Because of this whiteness, when added to a food product, the solid material does not change a color of the food product, which is preferred.

The specification of Japanese Patent Application No. 2011-27216 filed by Mitsui Sugar. Co., Ltd., one of the present applicants, describes a process for producing a solidified material from a saccharide solution comprising isomaltulose, wherein a sucrose solution is subjected to action of an enzyme which produces isomaltulose from sucrose to prepare the isomaltulose-containing saccharide solution. The solid material obtained by that process is not spherical. In contrast, the isomaltulose-containing solidified material obtained by the present process is spherical.

### BRIEF DESCRIPTION OF THE DRAWINGS

Fig.1 is a copy of a photograph of the isomaltulose-containing solid material obtained by the present process, which photograph was taken by a digital microscope.
Fig.2 is a schematic illustration of the isomaltulose-containing solid material obtained by the present process.

### EMBODIMENTS OF THE INVENTION

In the present invention, "isomaltulose" is a di-saccharide in which glucose is bound to fructose via an alpha-1,6-glucosyl bond. Isomaltulose is called also as Palatinose (trademark), and hereinafter referred to as Palatinose.

In the present invention, "an enzyme which produces isomaltulose from sucrose" may be any enzyme as long as the enzyme generates isomaltulose from sucrose. The enzyme is, for example, an alpha-glucosyltransferase. The alpha-glucosyltransferase is derived from, for example, Protaminobacter rubrum, Serratia plymuthica, Erwinia rhapontici, or Klebsiella sp..

In the present invention, "a saccharide solution comprising isomaltulose" refers to one obtained by subjecting a sucrose solution to action of an enzyme which produces isomaltulose from sucrose. The sucrose solution may be such for the enzyme to produce isomaltulose. For example, the sucrose solution may be a raw liquor, a brown liquor, a carbonated liquor, and a fine liquor which are obtained in sugar production. The sucrose solution may comprise sucrose in an amount of 5 to 60 mass%, especially 10 to 50 mass%, for optimal enzyme reaction. The sucrose solution may comprise saccharides other than sucrose. It is preferred that the amount of sucrose is 97 mass% or more, based on a total mass of all saccharides contained in the sucrose solution. The sucrose solution may be subjected to the action of the enzyme according to the method described in Japanese Patent Application Laid-Open No. SHO-57-39794, but not limited to it. The saccharide solution comprising isomaltulose is obtained by subjecting the sucrose solution to the action of the enzyme. The saccharide solution obtained by subjecting a sucrose solution to action of the enzyme (hereinafter, referred to as "isomaltulose-containing saccharide solution") comprises other saccharides besides isomaltulose. Such other saccharides results from the aforesaid enzyme action, such as trehalulose, fructose, glucose, sucrose, isomaltose, and isomelezitose. The isomaltulose-containing saccharide solution may comprise minerals and/or amino acids. The isomaltulose-containing saccharide solution may further comprise other components. Such components are added, for example, to keep concentrations of the components contained in the saccharide solution constant among batches. In the present process, kinds and concentrations of saccharides in the isomaltulose-containing saccharide solution is determined by a routine manner in the present technical field, such as high-performance liquid chromatography.

Isomaltulose accounts for 70 to 90 mass%, preferably 72 to 89 mass%, more preferably 74 to 88 mass%, even more preferably 75 to 85 mass%, of saccharides contained in the isomaltulose-containing saccharide solution. In calculating this isomaltulose percentage, a denominator is a total mass of isomaltulose, trehalulose, fructose, glucose, sucrose, and isomaltose contained in the isomaltulose-containing saccharide solution. A mass of a saccharide is calculated as that of its anhydride. When the percentage of isomaltulose is too low, the saccharide solution can not be solidified. The percentage of isomaltulose may be higher than the aforesaid upper limit, but generally the percentage is determined, depending upon an isomaltulose percentage in the saccharide solution resulting from the enzyme action in view of production efficiency, but not exceeding the aforesaid upper limit.

The mass percentage of trehalulose, relative to the total mass of saccharides contained in the isomaltulose-containing saccharide solution is 8 to 25 mass%, particularly 9 to 20 mass%, more particularly 10 to 18 mass%. The mass percentage of glucose, relative to the total mass of saccharides contained in the isomaltulose-containing saccharide solution, may be, for example, 0.1 to 5 mass%, particularly 0.2 to 4 mass%, more particularly 0.3 to 3 mass%. The mass percentage of fructose, relative to the total mass of saccharides contained in the isomaltulose-containing saccharide solution, may be, for example, 0.1 to 5 mass%, particularly 0.2 to 4 mass%, more particularly 0.3 to 3 mass%. Also in calculating these percentages, a denominator is a total mass of isomaltulose, trehalulose, fructose, glucose, sucrose, and isomaltose contained in the saccharide solution, as in calculating the mass percentage of isomaltulose. Masses of these saccharides are calculated as those of these anhydrides. One or more of the percentages of these saccharides other than isomaltulose is believed to contribute to the resulting shape and dryness of the solid material described below. Especially, the combination of the percentages of isomaltulose and trehalulose is believed to contribute to such.

The isomaltulose-containing saccharide solution may be in any form. For example, isomaltulose and saccharides other than isomaltulose may be dissolved, suspended or dispersed in the solution, or precipitated in the solution. For example, the saccharide solution is a water containing isomaltulose and saccharides other than isomaltulose.

In the present invention, the isomaltulose-containing solid material comprises isomaltulose and a non-crystalline saccharide solution. In the isomaltulose-containing solid material, isomaltulose is especially solid. Further, the present solid material comprises isomaltulose crystals. The non-crystalline saccharide solution is believed to consist mainly of trehalulose. The non-crystalline saccharide solution may further comprise saccharides other than trehalulose. Among saccharides comprised in the non-crystalline saccharide solution, saccharides are believed not to be crystal. For example, trehalulose is non-crystalline. Saccharides present in low contents, such as glucose, fructose, and sucrose, are believed to be in a syrup and not to be crystallized. The non-crystalline saccharide solution especially comprises trehalulose, fructose, glucose, sucrose, isomaltose, and/or isomelezitose. The non-crystalline saccharide solution may also comprise isomaltulose. The composition of saccharides in the isomaltulose-containing solid material is same as that in the aforesaid isomaltulose-containing saccharide solution. For example, the mass percentage of isomaltulose (including isomaltulose crystals), relative to the total mass of the solid material is 70 to 90 mass%, more especially 72 to 89 mass%, even more especially 74 to 88 mass% or 75 to 85 mass%. In the present solid material, the non-crystalline saccharide solution may be liquid wholly, or a part of the solution may be liquid and the remaining part may form solid. Especially, trehalulose accounts for 40 to 99 mass%, particularly 45 to 80 mass%, more particularly 50 to 70 mass%, of the mass of the non-crystalline saccharide solution. Especially, isomaltulose in the non-crystalline saccharide solution accounts for 3 to 30 mass%, particularly 5 to 25 mass%, more particularly 7 to 20 mass%, of the mass of the non-crystalline saccharide solution.

The present solid material may be especially in a form of a spherical particle. In the present invention, a spherical particle may not be exactly spherical, but may substantively spherical. The spherical form includes a form of an oval sphere and a form of a sphere having an irregular surface. Most of the particles have a diameter of 0.3 to 300 µm and some of the particles may have a diameter larger than the upper limit. The median diameter of the spherical particles is 60 to 300 µm, more preferably 80 to 200 µm, as determined by laser diffraction particle size distribution measurement. The particles may be in a form in which several particles are attached to each other.

The present solid material has voids. The voids are confirmed by the fact that when the solid material is dissolved in water, bubbles occur. It is believed that because of the voids, the present solid material has a high dissolution rate.

In the spherical particles, the non-crystalline saccharide solution is believed to be enveloped in the solid isomaltulose.

The present process will be described below in detail.

In the present process, isomaltulose is crystallized to obtain crystals having a median diameter of 5 to 60 µm, preferably 6 to 55 µm, more preferably 8 to 50 µm, in the isomaltulose-containing saccharide solution. The median diameter is determined by laser diffraction particle size diameter distribution measurement. For example, SALD-2000J (SHIMADZU CORPORATION) may be used for the measurement.

In the present invention, when the median diameter of the isomaltulose crystals obtained in the isomaltulose-containing saccharide solution is in the aforesaid range, the solidification, especially powderization, of the isomaltulose-containing saccharide solution is achieved. If the median diameter is larger than the upper limit, crystals and the non-crystalline saccharide solution are separated from each other in the spray-drying. As a result of the separation, the non-crystalline saccharide solution in a product of the spraydrying is not enveloped with solid isomaltulose (especially isomaltulose crystals), but rather the solid isomaltulose is enveloped with the non-crystalline saccharide solution. Then, such a product is highly hygroscopic and sticky and tends to aggregate. If the median diameter is smaller than the lower limit, the surface area of the crystals is too large. Consequently, a surface tension makes it difficult for the non-crystalline saccharide solution to flow, thus makes the viscosity of the liquid-crystal mixture high. Because of the high viscosity, spray-drying cannot be carried out.

In the present invention, isomaltulose may properly be crystallized according to any techniques known to a person having ordinary skill. Especially, a Brix of the isomaltulose-containing saccharide solution is adjusted and, then, the saccharide solution is aged, so that isomaltulose crystals occur in the saccharide solution. The adjustment of a Brix and the aging are described in detail in 1) and 2) below.

### 1) Adjustment of a Brix

A Brix of the isomaltulose-containing saccharide solution obtained by subjecting a sucrose solution to action of an enzyme which produces isomaltulose from sucrose is usually about 20 to 60 degrees, especially 30 to 50 degrees. This saccharide solution may be subjected directly to an aging step to cause isomaltulose to crystallize to obtain isomaltulose crystals in the solution. For efficient crystallization, it is preferred to adjust a Brix of the isomaltulose-containing saccharide solution to 50 to 80 degrees, more preferably 53 to 75 degrees, further more preferably 55 to 70 degrees, even more preferably 60 to 70 degrees. When the Brix is in the range, crystallization in the aging step is carried out more efficiently and a time necessary for the crystallization is shortened. The adjustment of a Brix is preferably carried out by heating in order to carry out the crystallization step more efficiently, but may be carried out by any other methods. The heating may be carried out in a routine manner known in the present technical field. For example, the saccharide solution is placed in a vessel, and heated by a heater with stirring. As examples of the heater, an evaporator, a vacuum pan, an effect evaporator, and a thin-film evaporator may be mentioned. By this heating in the present process, the temperature of the saccharide solution may be raised especially 100 to 115 degrees Celsius, more especially 102 to 111 degrees Celsius, even more especially 103 to 108 degrees Celsius, so that the Brix is adjusted in the aforesaid range. This heating may be conducted at normal pressure. In order to achieve such a desired Brix quickly, the heating may be conducted at a reduced pressure. In such a case, the saccharide solution is heated at 50 to 60 degrees Celsius, especially 52 to 59 degrees Celsius, more especially 53 to 57 degrees Celsius, and 100 mmHg to attain the aforesaid Brix.

### 2) Aging

In the present invention, the aging means that the saccharide solution is kept in a certain temperature range in a certain time period. By the aging, isomaltulose crystals grow in the saccharide solution. The aging may be carried out in a crystallizer or a refrigerator. As examples of the crystallizer, a tank equipped with a stirrer and a jacket or coil for cooling water, a vertical crystallizer, a ribbon mixer may be mentioned. A time period necessary for the aging is, for example, 12 to 48 hours at 20 to 40 degrees Celsius. Before the aging, a step of increasing the number of crystals may be carried out. This step is carried out by, for example, adding a small amount, such as 0.01 to 0.5 %DS (solid mass percentage), of seed crystals having a median diameter of 50 micrometers or less to the saccharide solution having the adjusted Brix and stirring the saccharide solution by a high-speed rotation homogenizer at 8000 to 20000 rpm, so that a larger number of crystals than that of the added seed crystals occur in the saccharide solution. In the aging, the obtained crystals act as nuclei for growing. Instead of the step of increasing the number of crystals, 0.1 to 5 %DS of seed crystals having a median diameter of 25 micrometers or less are added to the saccharide solution having the adjusted Brix and, then, the crystals are dispersed by a stirrer at 200 to 1500 rpm. In the aging, the dispersed crystals act as nuclei for growing. The seed crystals may be, for example, those obtained by crushing commercially-available isomaltulose crystals by a hammer mill. As the hammer-mill, Sample Mill KIIW-1 (Fuji Paudal Co., Ltd.) may be mentioned, but not limited to it. The seed crystals may be, for example, a slurry containing isomaltulose crystals.

A yield of crystals obtained by the aging depends on a Brix of the saccharide solution. For example, about a half of the mass of the solid content contained in the saccharide solution may be crystallized. The yield is preferably 30 to 80 mass%, more preferably 40 to 70 mass%. For determining the yield, 1 g of the saccharide solution containing crystals is placed in a 1.5 ml-volume eppendorf tube and centrifuged by a centrifugal separator (M150IV, Sakuma Seisakusho Co., Ltd.) at 16,000 rpm for one minute, a supernatant is decanted, and the yield is calculated from the mass of the crystals.

A viscosity of the saccharide solution after the aging may be such at which the saccharide solution can be spray-dried by a spraydryer, such as, for instance, 4000 mPa·s or less, more especially 2000 mPa·s or less, and even more especially 1000 mPa·s or less. The viscosity is determined, for example, by a rotational viscometer. For example, clogging is avoided at this viscosity in the subsequent spray-drying step, so that better spraying is attained. A specific gravity of the saccharide solution after the aging is preferably 1 to 1.7 g/ml, more preferably 1.05 to 1.65 g/ml, even more preferably 1.1 to 1.6 g/ml. The specific gravity of the saccharide solution is determined, for example, by a specific gravity bottle (Gay-Lussac type). Better spraying is attained at this specific gravity in the subsequent spray-drying step.

In the preset invention, the saccharide solution is spray-dried to obtain the isomaltulose-containing solid material of the present invention. An air temperature in the spraydrying is 50 to 95 degrees Celsius, preferably 53 to 93 degrees Celsius, more preferably 55 to 90 degrees Celsius. If the temperature is lower than the lower limit, the saccharide solution is not sufficiently dried, so that solidification is insufficient. When the temperature is in this range, the aforesaid particle structure is attained, so that non-sticky, dry solid material, especially powder, is obtained. If the temperature is higher than the upper limit, a color of the resulting product is brown. Addition of such a brown product to a food alters a color of the food. Therefore, such a brown isomaltulose product is not preferred. The brown product has odor and cannot be commercially sold. This browning is thought to be due to scorching of saccharides (caramelization). When the temperature is in the aforesaid temperature range, a white isomaltulose-containing solid material is obtained.

In the present invention, the air temperature is a temperature at an inlet for the air to be introduced into a drying chamber of a spraydryer. The air temperature is measured by a temperature sensor usually attached to a spraydryer. A location to which the temperature sensor is attached is generally near a part for connecting a drying chamber with a conduit for introducing the hot air.

In the present invention, conditions other than the air temperature in the spraydrying step may be properly set. Especially, those conditions are as follows. An apparatus for spraydrying may be a usual spraydryer or other apparatus having a spraydrying function equivalent to that of a spraydryer. A size or internal diameter of a drying chamber of a spraydryer may be, for example, 300 to 5000 mm. This size of the drying chamber may be set depending on a production scale of the isomaltulose-containing solid material. The spraying is carried out, for example, by a nozzle or an atomizer. An atomizer is especially preferred because of the viscosity of the saccharide solution and because of the fact that the saccharide solution contains solid materials. The atomizer may be of a pressure type, a rotating type, or combination of them. A rotary atomizer is preferred in order to avoid clogging of a pump or a nozzle with the saccharide solution. As an atomizer disk, a pin-type disk may be mentioned, but not limited to it. A diameter and a rotation speed of the atomizer disk may be properly set depending on a size of a drying chamber of a spraydryer. A feeding rate of the saccharide solution may be preferably 2 to 15 kg/Hr, more preferably 2.5 to 14 kg/Hr, even more preferably 3 to 13 kg/Hr. If the rate is too low, a production rate is low. If the feeding rate is too high, clogging with the saccharide solution may occur or insufficient drying in the spry-dried product may be occur. A tube for feeding the massecuite may be, for example, a silicone tube having an internal diameter of 3 to 8 mm and an external diameter of 6 to 14 mm. The massecuite is fed to a spraydryer through the tube. A feeding pump may be, for example, a roller pump. In the spraydrying, an outlet air temperature may be, for example, 30 to 70 degrees Celsius, especially 35 to 60 degrees Celsius. The outlet air temperature is set depending on conditions for spraydrying, such as a hot air temperature and a feeding rate of the massecuite. The outlet air temperature may be measured by a temperature sensor near an air outlet.

The isomaltulose-containing solid material obtained by the present process has a very high dissolution rate. In a condition where 80 g of distilled water is put in a 200 ml-volume beaker and kept at 20 degrees Celsius in a water bath having a temperature of 20 degrees Celsius, to which 20 g of the solid material is added with stirring by a magnetic stirrer at 400 rpm, a time period necessary for the solid material to dissolve may be 150 seconds or less, especially 130 seconds or less, more especially 110 seconds or less, even more especially 100 seconds or less. Dissolution rates of crystalline Palatinose IC and powdery Palatinose ICP are about 262 seconds and about 235 seconds, respectively. Thus, the dissolution rate of the solid material is much higher than those of commercially available Palatinose products. For example, when the solid material is added to a food product, workability is good because of the high dissolution rate.

A bulk specific gravity of the isomaltulose-containing solid material obtained by the present process may be 1.2 g/ml or less, more especially 1.1 g/ml or less, even more especially 1.0 g/ml or less, as determined by the method described in Example 9 below. Bulk specific gravities of crystalline Palatinose IC and powdery Palatinose ICP are, for example, 0.817 g/ml and 0.41 g/ml.

A water content of the isomaltulose-containing solid material obtained by the present process may be 0.5 to 4 mass%, especially 0.6 to 3.5 mass%, more especially 0.7 to 3 mass%, as determined by the method described in Example 9 below. Water contents of crystalline Palatinose IC and powdery Palatinose ICP are 0.16 mass% and 0.23 mass%. Crystalline water is not counted in these water contents.

The present invention also provides a solid material comprising isomaltulose of 70 to 90 mass% and non-crystalline saccharide solution, wherein the solid material is in a spherical form. The solid material is obtained by the aforesaid process.

The present invention will be further explained with reference to the Examples below, but not limited to these Examples.

In the following Examples, a Brix of the saccharide solution to be used as a raw material is calculated, based on a solid content of crystalline Palatinose IC or Palatinose ISK. The solid content of Palatinose ISK is 75 mass%. The solid content is a percentage of solids determined on the basis of a refractive index (refBrix). The percentage is determined by a refractometer. Because Palatinose crystals contain crystalline water of 5%, the solid content of Palatinose crystal is 95%. When Palatinose IC (solid content: 95%), Palatinose ISK (solid content: 75%) and water are mixed in a certain mass proportion, a solid content of the mixture is calculated from these solid contents and the proportion. The solid content of the mixture is a Brix of the saccharide solution to be used as a raw material.

A particle diameter in the following Examples is a median diameter, unless otherwise indicated. The particle diameter is determined by a laser diffraction particle size distribution measurement device (Laser Diffraction Particle Size Analyzer SALD-2000J, SHIMADZU CORPORATION).

### Example 1

Production of an Isomaltulose-Containing Solid Material A model solution for an isomaltulose-containing saccharide solution obtained by subjecting a sucrose solution to action of an enzyme which produces isomaltulose from sucrose was prepared by mixing crystalline Palatinose IC (Mitsui Sugar Co., Ltd.), Palatinose Syrup ISK (Mitsui Sugar Co. , Ltd.) and water in a mass proportion of IC: ISK:water=51:18:26 and dissolving solid contents. The saccharide composition of the model solution is shown in Table 1.

Table 1

**Table 1. Saccharide Composition of the Model Solution (mass%)**

| Palatinose | Trehalulose | Fructose | Glucose | Sucrose | Others |
|---|---|---|---|---|---|
| **82.7** | **11.2** | **2.5** | **2.5** | **0.4** | **0.7** |

An enzyme reaction solution for producing isomaltulose is demineralized and then subjected to a step of separating Palatinose crystals. The separated Palatinose crystals are Palatinose IC (Mitsui Sugar Co., Ltd.), and a syrup separated from the reaction solution is Palatinose Syrup ISK (Mitsui Sugar Co., Ltd.). The mixture of Palatinose IC (Mitsui Sugar Co., Ltd.) and Palatinose Syrup ISK (Mitsui Sugar Co., Ltd.) in the aforesaid proportion can be used as a model solution for an isomaltulose-containing saccharide solution obtained by subjecting a sucrose solution to action of an enzyme which produces isomaltulose from sucrose. The Brix of the model solution was about 65 degrees.

1 mass% of crushed Palatinose (median diameter: 13 micrometers, obtained by crushing Palatinose crystals (Mitsui Sugar Co., Ltd.) by a hammer mill) as seed crystals was added to the model solution, and the model solution was aged overnight at 20 degrees Celsius to crystallize isomaltulose in the model solution. The particle diameters of isomaltulose crystals contained in the model solution after the crystallization (hereinafter, referred to as "massecuite") were from 15 to 50 micrometers, and the median diameter of the crystals was 32.19 micrometers. The viscosity and the specific gravity of the massecuite were 100mPa·s and 1.28 g/ml, respectively. The viscosity was determined by a rotational viscometer. The specific gravity was determined by a specific gravity bottle (Gay-Lussac type). The massecuite was spray-dried. A spraydryer used was P260 (PRECI Co., Ltd.). The specification of the spraydryer was as follows: an internal diameter: 2600 mm, a type of spray: a rotary atomizer, a disk: pin-type disc (*φ* 100 mm), a size of the apparatus: *φ* 2600 mm, a tube for feeding the massecuite: one silicone tube (internal diameter: 6mm, external diameter: 10mm), a pump: RPSE2 type roller pump (middle position). The rotation speed of the atomizer was 12000 rpm. The feed rate of the massecuite to the spraydryer was 4.419 kg/hr. An air temperature (referred to also as "inlet temperature") was 85 degrees Celsius. An outlet air temperature was 67 to 68 degrees Celsius. As a result of the spraydrying, an isomaltulose-containing solid material (hereinafter, referred to as "the solid material of Example 1") was obtained.

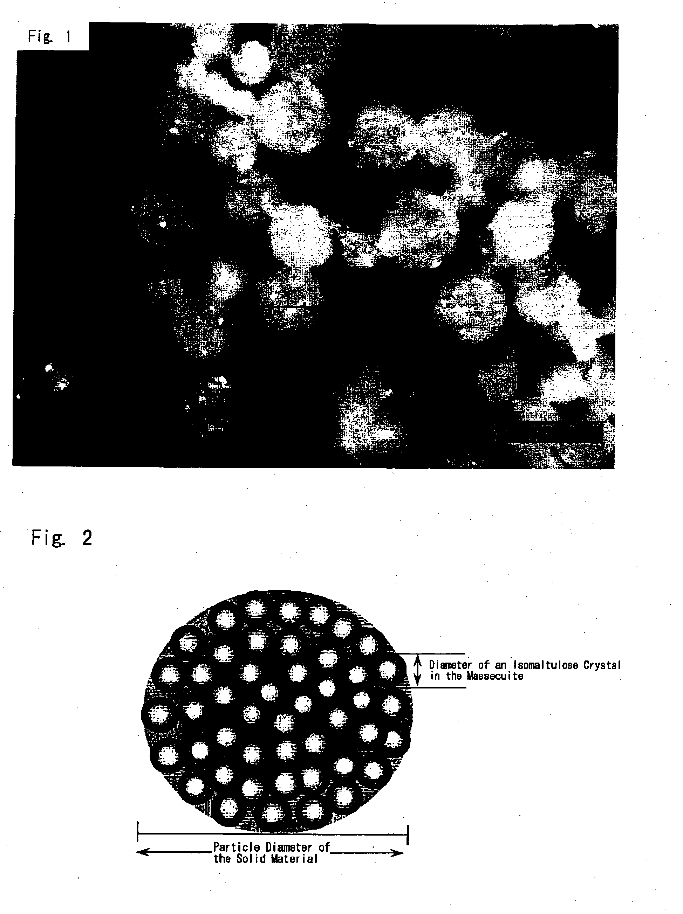

Fig.1 shows a copy of a photograph of the solid material, which photograph was taken by a digital microscope. One interval in the ruler in the photograph indicates 10 micrometers. The solid material was a spherical particle. The particle diameters of the solid material were about 22 to 500 micrometers. The median diameter of the solid material was about 131 micrometers. The solid material could be collected using a knocker or air. The collected solid material was non-sticky, dry powder. The solid material was white. Fig.2 shows a schematic illustration of a structure of the solid material. As shown in Fig.2, the solid material is believed to be a spherical particle in which isomaltulose crystals were contained in the massecuite aggregate. The isomaltulose crystals in the spherical particle are believed to be connected with each other via solid isomaltulose, and the non-crystalline saccharide solution is enveloped inside the particle.

### Example 2

The same procedures as in Example 1 were repeated except that the feed rate of the massecuite was 9.036 kg/hr, so that an isomaltulose-containing solid material (hereinafter referred to as "the solid material of Example 2") was obtained. The outlet air temperature was 66 degrees Celsius.

The solid material was a spherical particle. The particle diameters of the solid material were about 28 to 500 micrometers. The median diameter of the solid material was about 130 micrometers. The solid material could be collected using air. The collected solid material was non-sticky, dry powder. The solid material was white.

### Example 3

The same procedures as in Example 1 were repeated except that the air temperature was 90 degrees Celsius and the feed rate of the massecuite was 9.036 kg/hr, so that an isomaltulose-containing solid material (hereinafter referred to as "the solid material of Example 3") was obtained. The outlet air temperature was 68 to 69 degrees Celsius.

The solid material was a spherical particle. The particle diameters of the solid material were about 28 to 600 micrometers. The median diameter of the solid material was about 124 micrometers. The solid material could be collected using air. The collected solid material was non-sticky, dry powder. The solid material was white.

### Example 4

A massecuite was prepared in the same procedures as in Example 1 except that, before the aging, 10 g of Palatinose crystals were added as seeds to 1000g of the model solution produced in Example 1. The seeds were obtained by crushing Palatinose IC by a hammer mill (SAMPLE-MILL KIIW-I, Fuji Paudal Co., Ltd.). The viscosity and the specific gravity of the massecuite were 90 mPa·s and 1.304 g/ml, respectively.

The same procedures as in Example 1 were repeated except that this massecuite was spraydried, the hot air temperature was 90 degrees Celsius and the feed rate of the massecuite was 9.036 kg/hr, so that an isomaltulose-containing solid material (hereinafter referred to as "the solid material of Example 4") was obtained. The outlet air temperature was 69 to 71 degrees Celsius.

The solid material was a spherical particle. The particle diameters of the solid material were about 34 to 500 micrometers. The median diameter of the solid material was about 164 micrometers. The solid material could be collected using air. The collected solid material was non-sticky, dry powder. The solid material was white.

### Example 5

Palatinose IC (Mitsui Sugar Co. , Ltd.) and Palatinose Syrup ISK (Mitsui Sugar Co., Ltd.) were mixed in a mass ratio of 65:24. Table 2 shows the saccharide composition of the mixture.

Table 2

**Table 2. Saccharide Composition of the Model Solution (mass%)**

| Palatinose | Trehalulose | Fructose | Glucose | Sucrose | Others |
|---|---|---|---|---|---|
| **83.2** | **11** | **2.4** | **2.5** | **0.3** | **0.6** |

Water was added to the mixture, which was then heated to dissolve Palatinose IC, and its Brix was adjusted to 60.4 degrees. Then, the mixture was cooled to 25 degrees Celsius. After the cooling, 0.1%DS (solid mass percentage) of the crushed Palatinose as described in Examples 1 was added to the mixture. Then, the mixture was aged at 20 degrees Celsius for 2 hours in a crystallizer (Homogenizer ULTRA-TURRAX, IKA Works, Inc.) with stirring at 11000 rpm, so that a massecuite was obtained. As observed by an optical microscope, the diameters of isomaltulose crystal particles in the massecuite were 25 to 98 micrometers. The median diameter of the isomaltulose crystals in the massecuite was 32.5 micrometers.

The massecuite was spray-dried by a spraydryer (SA-5, Hirano Kogyo Co., Ltd.). The specification of the spraydryer was as follows: an internal diameter: 2000 mm, a type of spray: a centrifugal atomizer, a disk: a pin-type disc (*φ* 100 mm), a size of a drying chamber: *φ*2000 mm, a tube for feeding a massecuite: one silicone tube (internal diameter: 5 mm, external diameter: 8 mm), a type of pump: a roller pump (RP-2000). The rotation speed of the atomizer was 14000 rpm. The feed rate of the massecuite to the spraydryer was 4 kg/hr. The air temperature was 60 degrees Celsius. The outlet air temperature was 41 degrees Celsius. As a result of the spraydrying, an isomaltulose-containing solid material (hereinafter referred to as "the solid material of Example 5") was obtained.

The solid material was a spherical particle. The particle diameters of the solid material were about 22 to 500 micrometers, as observed by an optical microscope. The median diameter of the solid material was about 131 micrometers. The collected solid material was non-viscose, dry powder. The solid material was white.

### Example 6

The massecuite was prepared in the same procedures as in Example 5 except that the Brix was adjusted to 59.8 degrees. The diameters of the isomaltulose crystal particles in the massecuite were 3 to 25 micrometers, as observed by an optical microscope. The median diameter of the isomaltulose crystals was 17.7 micrometers.

The massecuite was subjected to the same spraydrying as in Example 5 except that the air temperature was 80 degrees Celsius. The outlet air temperature was 46 degrees Celsius. As a result of the spraydrying, an isomaltulose-containing solid material (hereinafter referred to as "the solid material of Example 6") was obtained.

The solid material was a spherical particle. The particle diameters of the solid material were 28 to 600 micrometers. The median diameter of the solid material was about 130 micrometers. The solid material was white. The collected solid material was non-viscose, dry powder.

### Example 7.

The masseucuite was prepared in the same procedures as in Example 5 except that the Brix was adjusted to 59.6 degrees. The diameters of the crystalline isomaltulose particles in the massecuite were 20 to 67 micrometers. The median diameter of the isomaltulose crystals was 25.7 micrometers.

The massecuite was subjected to the same spraydrying as in Example 5. The outlet air temperature was 37 degrees Celsius. As a result of the spraydrying, an isomaltulose-containing solid material (hereinafter referred to as "the solid material of Example 7") was obtained.

The solid material was a spherical particle. The particle diameters of the solid material were 28 to 600 micrometers, as observed by an optical microscope. The median diameter of the solid material was about 124 micrometers. The solid material was white. The collected solid material was non-viscose, dry powder.

### Example 8

The massecuite was prepared in the same procedures as in Example 5 except that the Brix was adjusted to 60. 6 degrees, the stirring speed was 16000 rpm and the cooling temperature was 10 degrees Celsius. The diameters of the crystalline isomaltulose particles in the massecuite were 1 to 25 micrometers, as observed by an optical microscope. The median diameter of the isomaltulose crystals was 15.4 micrometers.

The massecuite was subjected to the same spraydrying as in Example 5. The outlet air temperature was 42 degrees Celsius. As a result of the spraydrying, isomaltulose-containing solid material (hereinafter referred to as "the solid material of Example 8") was obtained.

The solid material was a spherical particle. The particle diameters of the solid material were 34 to 500 micrometers, as observed by an optical microscope. The median diameter of the solid material was about 164 micrometers. The solid material was white. The collected solid material was non-viscose, dry powder.

### Comparative Example 1

The massecuite was prepared in the same procedures as in Example 5 except that the Brix was adjusted to 51.2 degrees and the stirring speed was 120 rpm. The diameters of the crystalline isomaltulose particles in the massecuite were 150 to 156 micrometers, as observed by an optical microscope. The median diameter of the isomaltulose crystals was 120.2 micrometers.

The massecuite was subjected to the same spraydrying as in Example 5 except that the air temperature was 65 degrees Celsius. The outlet air temperature was 41 degrees Celsius. The product from the spraydrying was not dry powder, but sticky to adhere to the inside of the spraydrier. Thus, the isomaltulose and the non-crystalline saccharide solution were separated from each other in the product. Not the whole massecuite could be solidified. The product cannot be sold as such because of its stickiness.

### Comparative Example 2

The massecuite was prepared in the same procedures as in Example 5 except that the Brix was adjusted to 60.1 degrees and the stirring speed was 120 rpm. The diameters of the crystalline isomaltulose particles in the massecuite were 105 to 156 micrometers, as observed by an optical microscope. The median diameter of the isomaltulose crystals was 133.1 micrometers.

The massecuite was subjected to the same spraydrying as in Example 5 except that the hot air temperature was 65 degrees Celsius. The outlet air temperature was 41 degrees Celsius. The product from the spraydrying was not dry powder, but sticky to adhere to the inside of the spraydrier. Thus, the isomaltulose and the non-crystalline saccharide solution were separated from each other in the product. Not the whole massecuite could be solidified. The product cannot be sold as such because of its stickiness. Most of the solidified part in the product was in a form of agglomerates.

### Comparative Example 3

The massecuite was prepared in the same procedures as in Example 5 except that the Brix was adjusted to 60.2 degrees and the stirring speed was 120 rpm. The diameters of the crystalline isomaltulose particles in the massecuite were 45 to 207 micrometers, as observed by an optical microscope. The median diameter of the isomaltulose crystals was 103.6 micrometers.

The massecuite was subjected to the same spraydrying as in Example 5. The outlet air temperature was 41 degrees Celsius. The product from the spraydrying was not dry powder, but sticky to adhere to the inside of the spraydrier. Thus, the isomaltulose and the non-crystalline saccharide solution were separated from each other in the product. Not the whole massecuite could be solidified. The product cannot be sold as such because of its stickiness. Most of the solidified part in the product was in a form of agglomerates.

Table 3 below lists the conditions and the results of Examples 5 to 8 and Comparative Examples 1 to 3. In the row of the Results, G indicates that the whole isomaltulose-containing saccharide solution was solidified. B indicates that not the whole isomaltulose-containing saccharide solution was solidified. The solid materials obtained in Examples 5 to 8 were non-sticky, dry powder.

Table 3

**Table 3. Conditions and Results**

| | Example 5 | Example 6 | Example 7 | Example 8 |
|---|---|---|---|---|
| Median Diameter of Crystals in the Massecuite (*µ*m) | 32.5 | 17.7 | 25.7 | 15.4 |
| Air Temperature (°C) | 60 | 80 | 60 | 60 |
| Result | G | G | G | G |
| | | | | |

| | Comp. Ex. 1 | Comp. Ex. 2 | Comp. Ex. 3 | |
|---|---|---|---|---|
| Median Diameter of Crystals in the Massecu i te (*µ*m) | 120.2 | 133.1 | 103.6 | |
| Air Temperature (°C) | 65 | 65 | 60 | |
| Result | B | B | B | |

### Example 9

The solid materials of Examples 1 and 5 and two commercially available Palatinoses (crystalline Palatinose IC (Mitsui Sugar Co., Ltd., hereinafter referred to as "IC") and powdery Palatinose ICP (Mitsui Sugar Co. Ltd., hereinafter referred to as "ICP") were provided. Dissolution rates, bulk specific gravities and water contents of these four samples were determined.

### 1) Dissolution Rate

80 ml of distilled water was placed in a 200 ml-volume beaker and kept at 20 degrees Celsius in a water bath. While stirring by a magnetic stirrer at 400 rpm, 20 g of the sample was added to the water and the time (sec) necessary for the sample to dissolve completely was recorded. The results are shown in Table 4 below.

Table 4

**Table 4. Dissolution Rate**

| | 1st | 2nd | 3rd | Average ± SD |
|---|---|---|---|---|
| Example 1 | 24 | 27 | 24 | 25±1.7 |
| Example 5 | 78 | 77 | 72 | 76±3 |
| IC | 254 | 267 | 266 | 262±7 |
| ICP | 201 | 246 | 258 | 235±30 |

The dissolution rates of the solid materials of Examples 1 and 5 were much higher than those of crystalline Palatinose IC and Palatinose ICP. Bubbles occurred when the solid materials of Examples 1 and 5 were dissolved in water. These bubbles are due to the voids in the solid materials. No bubble occurred when IC and ICP were dissolved.

### 2) Bulk Specific Gravity

A bulk specific gravity was determined by ABD Powder Tester (TSUTSUI SCIENTIFIC INSTRUMENTS CO., LTD.), according to the instruction attached to the tester. A sample vessel (100 ml volume) was heaped with a sample taken from a hopper of the tester. Then, the sample above the upper tip of the vessel was removed. The remaining sample was weighed. A bulk specific gravity is calculated according to the following formula: Bulk Specific Gravity(g/ml) = Sample Mass (g) / 100 (ml). The results are shown in Table 5 below.

Table 5

**Table 5. Bulk Specific Gravity (g/ml)**

| | |
|---|---|
| Example 1 | 0.703 |
| Example 5 | 0.665 |
| IC | 0.817 |
| ICP | 0.41 |

The specific gravities of the solid materials of Examples 1 and 5 were smaller than that of crystalline Palatinose IC.

### 3) Water Content

A water content was determined on the basis of a deference between masses before and after drying at 75 degrees Celsius and a reduced pressure for 3 hours. The results are shown in Table 6.

Table 6

**Table 6. Water Content (%wt)**

| | |
|---|---|
| Example 1 | 2.16 |
| Example 5 | 1.23 |
| IC | 0.16 |
| ICP | 0.23 |

### Example 10

A sucrose solution of 40 mass% was subjected to action of alpha-glucocyltranspherase derived from Protaminobacter rubrum to obtain an isomaltulose-containing saccharide solution. Then, the isomaltulose-containing saccharide solution was demineralized. The enzyme reaction and the demineralization were carried out according to the methods described in "Manufacture and Utilization of Palatinose", Yoshikazu Nakajima, Journal of the Japanese Society of Starch Science, Japanese Society of Starch Science, 1988, Vol.35, No.2, Pages 131 to 139. The Brix of the demineralized solution was 38.2 degrees. The saccharide composition of the demineralized solution was as shown in Table 7.

Table 7

**Table 7. Saccharide Composition of the Demineralized Solution (mass%)**

| Palatinose | Trehalulose | Fructose | Glucose | Sucrose | Others |
|---|---|---|---|---|---|
| **83.61** | **10.8** | **2.2** | **1.9** | **1.5** | **0** |

The demineralized solution was heated at 85 degrees Celsius in a 10 litter-volume flask of a rotary evaporator (N-11, TOKYO RIKAKIKAI CO., LTD.) connected to a cooling trap (UT-50, TOKYO RIKAKIKAI CO., LTD.) and a diaphragm vacuum pump (DIVAC2.2L, TOKYO RIKAKIKAI CO., LTD.), so that a concentrated solution having a Brix of 63 degrees was obtained. The concentrated solution was cooled to 25 degrees Celsius while stirring. 70 g of Crushed Palatinose (median diameter: 15 micrometers, crystalline Palatinose crushed by a hammer mill) was added to 7 kg of the concentrated solution at 25 degrees Celsius, and aged overnight at 25 degrees Celsius to crystallize isomaltulose to obtain crystals having a diameter of 15 to 50 micrometer, so that a massecuite was obtained. The median diameter of the crystals in the massecuite was 35.2 micrometers. The viscosity and the specific gravity of the massecuite after the aging were 120 mPa·s and 1.305 g/ml, respectively.

Using a spraydryer (small spraydryer R160, PRECI Co., Ltd.), the massecuite was spray-dried at an air temperature of 90 degrees Celsius, an atomizer rotation speed of 16000 rpm and a feed rate of the saccharide solution to the spraydryer of 1.13 kg/h. An outlet air temperature was 67 to 71 degrees Celsius. As a result of the spraydrying, an isomaltulose-containing solid material (hereinafter, referred to as "the solid material of Example 10") was obtained. The isomaltulose-containing solid material had a median diameter of about 124.4 micrometers. The solid material was spherical. The collected solid material was non-sticky, dry powder.

### Test Example 1: Production of Hard Candies and Evaluation

7 parts by mass of the solid material of Example 10 and 3 parts by mass of water were placed in a pan, and mixed and heated on a fire. When a liquid temperature of the mixture reached 160 degrees Celsius, the pan was removed from a fire. The liquid was poured into molds and solidified to obtain hard candies.

The same procedures as described above were repeated, except that Palatinose (crystalline Palatinose IC, Mitsui Sugar Co., Ltd.) was used instead of the solid material of Example 10.

The hard candies prepared from the solid material of Example 10 and from Palatinose IC were as transparent. When hard candies are prepared from sucrose only, crystals appears in a condensing or solidifying step, so that transparent hard candies are not obtained. It was shown that, when candies are prepared from Palatinose IC or the present solid material, transparent candies could be obtained.

### Test Example 2: Production of Yoghurt Drinks and Evaluation

### Production of Yoghurt Drinks

Yoghurt drinks were produced using the ingredients in the compositions of Tests 1 to 3 indicated in Table 8 below. The amounts of the Solid Material of the Example and Palatinose were 1/0.45 times as much as that of sucrose in order to provide the same degree of sweetness among them. Production procedures are as follows. (1) Powdered skim milk was added to yoghurt and mixed so that lumps are not formed. (2) Milk was added to the mixture obtained in (1) and mixed, and each saccharide was added and mixed. (3) A citric acid solution of 30 mass% was added to the mixture obtained in (2) to adjust a pH at 4.5 to obtain yoghurt drinks.

Table 8

**Table 8: Ingredient and Composition for Yoghurt Drink**

| Composition (g) | Test 1 | Test 2 | Test 3 |
|---|---|---|---|
| Yoghurt | **100** | **100** | **100** |
| Milk | **100** | **100** | **100** |
| Solid Material of Example 10 | **31** | **-** | **-** |
| Sugar (granulated sugar GN, (Mitsui Sugar Co., Ltd.) | **-** | **15** | **-** |
| Palatinose (crystalline PalatinoselC, Mitsui SugarCo., Ltd.) | **-** | **-** | **31** |
| Powdered Skim Milk | **1.5** | **1.5** | **1.5** |

| | | | |
|---|---|---|---|
| A 30% citric acid solution was added to attain a pH of 4.5. | | | |

### Evaluation of the Appearance

The color of the yoghurt drinks of Tests 1 to 3 were analyzed by a chromameter (CR-400, KONICA MINOLTA, INC.). The values in Table 9 are in accordance with a CIE color-difference formula L*a*b* defined by the International Commission on Illumination.

Table 9

**Table 9: Color Differences**

| | L* Value | a* Value | b* Value |
|---|---|---|---|
| Test 1 | 84.34 | -3.7 | 5.87 |
| Test 2 | 84.73 | -3.51 | 5.86 |
| Test 3 | 84.6 | -3.65 | 5.77 |

As seen in Table 9, almost no difference was found among the yoghurt drinks of Tests 1 to 3. Thus, using the solid material of Example 10, yoghurt drinks are produced which have the same appearance as those produced with sugar or Palatinose. Viscosities of the yoghurt drinks of Tests 1 and 3 were higher than that of Test 2. This is because the amounts of saccharide in Tests 1 and 3 were larger than that in Test 2.

### Evaluation of Taste

As described above, the degrees of sweetness were made equal among Tests 1 to 3. However, the yoghurt drink of Test 2 was sweetest, and the yoghurt drink of Test 3 was least sweet. Sweetness of Test 1 was weaker than that of Test 2, but could be sufficiently felt.

The yoghurt drink of Test 1 was sourest, while that of Test 2 was least sour.

Comparing the taste of the yoghurt drink of Test 3 with that of Test 1, the taste of Test 3 was simple and refreshing, whereas the yoghurt of Test 1 had rich sweetness and a rich taste.

### Test Example 3: Production of Whipped Creams and Evaluation

### Production of Whipped Creams

Whipped creams were produced, using the ingredients in the compositions of Tests 1 to 3 indicated in Table 10 below. The amounts of the solid material of Example 10 and Palatinose were 1/0.45 times as much as that of sucrose in order to provide the same degree of sweetness among them. Each saccharide was added to fresh cream and whipped by a hand mixer. The whipping was stopped at the time when the cream reached stiff-peak.

Table 10

**Table 10: Ingredient and Composition of Whipped Cream**

| Composition (g) | Test 1 | Test 2 | Test 3 |
|---|---|---|---|
| Fresh Cream | **100** | **100** | **100** |
| Solid Material of Example 10 | **16.67** | **-** | **-** |
| Sugar (powdery sugar, Mitsui Sugar Co., Ltd.) | **-** | **7.5** | **-** |
| Palatinose (powdery Palatinose ICP, Mitsui Sugar Co., Ltd.) | **-** | **-** | **16.67** |

No difference in texture and taste was recognized among the whipped creams of Tests 1 to 3. The whipped creams of Tests 1 and 3 became foamy earlier than that of Test 2.

### Test Example 4: Production of Chocolates and Evaluation

### Production of Chocolates

Chocolates were produced, using the ingredients in the compositions of Tests 1 to 3 indicated in Table 11 below. In Tests 1 and 2, half the amount of the sugar of Test3 compositions was replaced with solid material of Example 10 or Palatinose to supplement sweetness. Chocolates were produced as follows. First, the saccharide was crushed by a crusher and added to black chocolate and heated in a bowl in hot water. After the black chocolate melted, they were well mixed at 40 to 45 degrees Celsius for 5 minutes so that air was not entrapped in the chocolate. After the saccharide was mixed uniformly, the mixture was heated to 56 degrees Celsius and, then, cooled to 28 degrees Celsius by placing the bowl in cold water. Then, the mixture was heated to 31 degrees Celsius by placing the bowl in hot water, poured into molds, and cooled to obtain chocolates.

Table 11

**Table 11: Ingredients and Compositions of Chocolates**

| Composition (g) | Test 1 | Test 2 | Test 3 |
|---|---|---|---|
| Black Chocolate (Cacao 86%) | **40** | **40** | **40** |
| Sugar (granulated sugarGN. Mitsui Sugar Co., Ltd.) | **4.8** | **4.8** | **9.6** |
| Solid Material of Example 10 | **4.8** | **-** | **-** |
| Palatinose (crystalline Palatinose IC, Mitsui Sugar Co., Ltd.) | **-** | **4.8** | **-** |

### Evaluation of Taste

Compared to Test 2, the chocolate of Test 1 had richer sweetness and better smell and flavor. The chocolate of Test 3 was the sweetest, and that of Test 2 was least sweet.

The chocolate of Test 3 was bitterer than those of Tests 1 and 2. Because the chocolate of Test 2 was less sweet, only bitterness was strongly felt. The chocolate of Test 1 was very sweet and bitter, and the flavor of cacao was felt well.

### Test Example 5: Production of Ground-green-tea Sponge Cakes and Evaluation

### Production of Sponge Cakes

Sponge cakes were produced, using the ingredients in the compositions of Tests 1 to 3 indicated in Table 12 below. In Tests 1 and 2, half the amount of the sugar was replaced with material of Example10 or Palatinose to supplement sweetness. Sponge cakes were produced as follows. First, cake flour and ground green tea were mixed and sieved. Butter was melted. Saccharide was added to whole eggs and beaten for 12 minutes by a hand mixer, while keeping the temperature at 30 degrees Celsius by warming it in a bowl in hot water. To the beaten and foamed liquid egg, the aforesaid mixture of cake flour and ground green tea was added in three portions and mixed gently. Then, the melted butter was added, and gently mixed, but not kneaded, to obtain a sponge cake dough. The dough was poured on cooking paper in a tray, and baked at 200 degrees Celsius for 15 minutes to obtain a ground-green-tea sponge cake.

Table 12

**Table 12: Ingredients and Compositions of Ground Green Tea Sponge Cakes**

| Composition (g) | Test 1 | Test 2 | Test 3 |
|---|---|---|---|
| Whole Egg | **150** | **150** | **150** |
| Sugar (granulated sugar GN, Mitsui Sugar Co., Ltd.) | **45** | **45** | **90** |
| Solid Material of Example 10 | **45** | **-** | **-** |
| Palatinase (crystalline Palatinose IC, Mitsui Sugar Co., Ltd.) | **-** | **45** | **-** |
| Cake Flour | **90** | **90** | **90** |
| Ground Green Tea | **10** | **10** | **10** |
| Salt-Free Butter | **15** | **15** | **15** |

Among the produced ground-green-tea sponge cakes, the sponge cake of Test 1 had mild sweetness and a light taste.

### Test Example 6: Production of Baton de Chocolat (bar-shaped chocolates) and Evaluation

### Production of bar-shaped chocolates

Bar-shaped chocolates were produced, using the ingredients in the compositions of Tests 1 to 3 indicated in Table 13 below. In Tests 1 and 2, half the amount of the sugar was replaced with solid materials of Example10 or Palatinose to supplement sweetness. Bar-shaped chocolates were produced as follows. First, butter was kneaded to become creamy, to which the saccharide was added and mixed. Whole eggs were gradually added to the mixture. Then, sieved powder of a mixture of flour, almond powder, and cocoa powder was added to obtain a dough. The dough was squeezed from a pastry bag onto a tray, baked at 170 degrees Celsius for 15 minutes to obtain bar-shaped chocolates.

Table 13

**Table 13: Ingredients and Compositions of Baton de Chocolat**

| Composition (g) | Test 1 | Test 2 | Test 3 |
|---|---|---|---|
| Cake Flour | **120** | **120** | **120** |
| Salt-Free Butter | **120** | **120** | **120** |
| Sugar (granulated sugar GN, Mitsui Sugar co., Ltd.) | **40** | **40** | **80** |
| Solid Material of Example 10 | **40** | **-** | **-** |
| Palatinose (crystalline Palatinose IC, Mitsui Sugar Co., Ltd.) | **-** | **40** | **-** |
| Whole Egg | **50** | **50** | **50** |
| Almond Powder | **30** | **30** | **30** |
| Cocoa Powder | **20** | **20** | **20** |
| Water | **13.31** | **13.3** | **13-3** |

The chocolate of Test 1 had less light taste than that of Test 2, but had lighter taste than that of Test 3. The chocolate of Test 1 was the bitterest.

### Test Example 7: Production of Carrot Jellies and Evaluation

### Production of Carrot Jellies

Carrot Jellies were produced, using the ingredients in the compositions of Tests 1 to 3 indicated in Table 14 below. Carrot Jellies were produced as follows. First, carrots were cut into an appropriate size and, then, processed in a mixer together with water and a lemon juice (15 seconds, 3 times) to obtain a carrot juice. Separately, a gelling agent and the solid material of Example 10, Palatinose, or sugar was well mixed, and added to the carrot juice in a pan, well mixed, and heated to boil for 3 minutes. The boiled liquid was poured into a mold, and cooled to obtain a carrot jelly.

Table 14

**Table 14: Ingredients and Compositions of Carrot Jellies**

| Composition (g) | Test 1 | Test 2 | Test 3 |
|---|---|---|---|
| Carrot | **100** | **100** | **100** |
| Lemon Juice | **15** | **15** | **15** |
| Water | **250** | **250** | **250** |
| Solid Material of Example 10 | **60** | **-** | **-** |
| Palatinose (crystalline Palatinose IC, Mitsui Sugar Co., Ltd.) | **-** | **60** | **-** |
| Sugar (granulated SugarGN, Mitsui Sugar Co., Ltd.) | **-** | **-** | **60** |
| Agent (WP-501, Mitsui Sugar Co., Ltd.) | | **4** | **4** |
| Water | **13.3** | **13.3** | **13.3** |

The jellies of Tests 1 and 2 had less grassy smell of carrot than that of Test 3. Conventionally, Palatinose is added to mask the smell. It was shown that the solid material of Example 10 attain the masking effect like Palatinose.

### Test Example 8: Production of Strawberry Jams and Evaluation

### Production of Strawberry Jams

Strawberry jams were produced, using the ingredients in the compositions of Tests 1 to 3 indicated in Table 15 below. A proportion of sucrose to the solid material of Example 10 was varied to obtain a strawberry jam having a Brix of 60 degrees and no crystals. In order to prevent formation of crystals, trehalulose syrup (Mildear75, Mitsui Sugar Co., Ltd.) was added in an amount of 20 parts by mass per total 100 parts by mass of the whole saccharides for each Test.

Table 15

**Table 15: Ingredients and Compositions of Strawberry Jams**

| Composition (g) | Test 1 | Test 2 | Test 3 |
|---|---|---|---|
| Frozen Strawberry | **200** | **200** | **200** |
| Trehalulose Syrup | **20** | **20** | **20** |
| Sugar | **40** | **30** | **20** |
| Solid Material of Example 10 | **40** | **50** | **60** |

Crystals were formed in the jam of Test 3 after one-month storage in a refrigerator. No crystal was formed in the jams of Tests 1 and 2 after one-month storage in a refrigerator, nor even after freezed for three days, followed by thawing.

As to sweetness, the jam of Test 3 was not so sweet as that of Test 1, but was sufficiently sweet. The more amount of the solid material of Example 10 the jam contained, the lighter sweetness and the stronger sourness the jam had. The jam of Test 3 had good balance between the sweetness and the sourness, and gave a strong strawberry flavor.

### Test Example 9: Production of Chewing Gums and Evaluation

Chewing Gums were produced using Palatinose or the solid material of Example 10. A production method was as follows. First, 15 parts by mass of enzymatically-saccharified corn syrup (KosoSyrup H85C, Brix of 85 degrees, Nihon Cornstarch Corporation) was added to 30 parts by mass of a chewing gum base, and kneaded for 5 minutes by a kneader (Benchkneader PNV-1, IRIE SHOKAI Co., Ltd.) which was warmed at 45 degrees Celsius. To the resulting mixture, 54 parts by mass of Palatinose (powdery Palatinose ICP, Mitsui Sugar Co., Ltd.) or of the solid material of Example 10 was added in several portions and, then, 1 part by mass of glycerol (JUNSEI CHEMICAL CO., LTD., food additive grade) was added, and kneaded for 15 minutes. Then, 1 part by mass of a flavor (peppermint oil, TAKATA KORYO CO., LTD.) was added to the mixture and, then, kneaded for 5 minutes. Then, the kneaded material was rolled into a stick gum (thickness of 2mm, width of 2 cm, length of 7 cm), using Palatinose (powdery Palatinose ICP, Mitsui Sugar Co. , Ltd.) to prevent the material from sticking, wrapped with an aluminum foil to obtain a chewing gum.

The resulting gums were examined one week after the production. The chewing gum produced with the solid material of Example 10 was softer than that produced with Palatinose.

## Claims

1. A process for producing a solid material from a saccharide solution comprising isomaltulose, wherein said solid material comprises isomaltulose of an amount of 70 to 90 mass% and trehalulose of an amount of 8 to 25 mass%, wherein a sucrose solution is subjected to action of an enzyme which produces isomaltulose from sucrose to prepare said saccharide solution comprising isomaltulose, **characterized in that** the process comprises steps;
causing isomaltulose to crystallize to obtain crystals having a median diameter of 5 to 60 µm in said saccharide solution, wherein the median diameter is determined by laser diffraction particle size distribution measurement, and
spray-drying the saccharide solution containing the isomaltulose crystals at an air temperature of 50 to 95 degrees Celsius to obtain said solid material.

2. The process according to claim 1, wherein a Brix of the saccharide solution is adjusted to 50 to 80 degrees before said crystallization.

3. A solid material comprising isomaltulose of an amount of 70 to 90 mass% and trehalulose of an amount of 8 to 25 mass%, wherein said solid material is in a spherical form and has a median diameters of 60 to 300 µm, as determined by laser diffraction particle size distribution measurement.

4. The solid material according to claim 3, wherein trehalulose is enveloped in the solid isomaltulose.

## Patentansprüche

1. Verfahren zur Herstellung eines Feststoffes aus einer Saccharidlösung, umfassend Isomaltulose, wobei der Feststoff Isomaltulose in einer Menge von 70 bis 90 Masse-% und Trehalulose in einer Menge von 8 bis 25 Masse-% umfasst, wobei eine Sucroselösung der Wirkung eines Enzyms, das Isomaltulose aus Sucrose erzeugt, unterzogen wird, um die Isomaltulose umfassende Saccharidlösung herzustellen, **dadurch gekennzeichnet, dass** das Verfahren Schritte umfasst:
Kristallisierenlassen von Isomaltulose, um Kristalle mit einem mittleren Durchmesser von 5 bis 60 µm in der Saccharidlösung zu erhalten, wobei der mittlere Durchmesser durch Laserdiffraktion-Teilchengrößenverteilungsmessung bestimmt wird, und
Sprühtrocknen der Isolmaltulosekustalle enthaltenden Saccharidlösung bei einer Lufttemperatur von 50 bis 95 Grad Celsius, um den Feststoff zu erhalten.

2. Verfahren nach Anspruch 1, wobei ein Brix der Saccharidlösung auf 50 bis 80 Grad vor der Kristallisation eingestellt wird.

3. Feststoff, umfassend Isomaltulose in einer Menge von 70 bis 90 Masse-% und Trehalulose in einer Menge von 8 bis 25 Masse-%, wobei der Feststoff in einer sphärischen Form vorliegt und einen mittleren Durchmesser von 60 bis 300 µm aufweist, wie bestimmt durch Laserdiffraktion-Teilchengrößenverteilungsmessung.

4. Feststoff nach Anspruch 3, wobei Trehalulose in der festen Isomaltulose eingehüllt ist.

## Revendications

1. Procédé pour produire un matériau solide à partir d'une solution de saccharide comprenant de l'isomaltulose, ledit matériau solide comprenant de l'isomaltulose en une quantité de 70 à 90 % en masse et du tréhalulose en une quantité de 8 à 25 % en masse, dans lequel une solution de saccharose est soumise à l'action d'une enzyme qui produit de l'isomaltulose à partir de saccharose pour préparer ladite solution de saccharide comprenant de l'isomaltulose, **caractérisé en ce que** le procédé comprend les étapes :
faire cristalliser l'isomaltulose pour obtenir des cristaux ayant un diamètre médian de 5 à 60 µm dans ladite solution de saccharide, ledit diamètre médian étant déterminé par mesure de distribution de granulométrie par diffraction laser, et
sécher par pulvérisation la solution de saccharide contenant les cristaux d'isomaltulose à une température de l'air de 50 à 95°C, pour obtenir ledit matériau solide.

2. Procédé selon la revendication 1, dans lequel le Brix de la solution de saccharide est ajusté à 50 à 80 degrés avant ladite cristallisation.

3. Matériau solide comprenant de l'isomaltulose en une quantité de 70 à 90 % en masse et du tréhalulose en une quantité de 8 à 25 % en masse, lequel matériau solide étant sous une forme sphérique et ayant un diamètre médian, tel que déterminé par mesure de distribution de granulométrie par diffraction laser, de 60 à 300 µm.

4. Matériau solide selon la revendication 3, dans lequel le tréhalulose est enveloppé dans l'isomaltulose solide.
